# EUROPEAN PATENT SPECIFICATION

(11) **EP 1 419 791 B1**
(45) Date of publication and mention of the grant of the patent: **24.02.2010**
(21) Application number: 02751674.9
(22) Date of filing: 25.07.2002
(51) Int. Cl.: A61L 27/00, A61L 27/60

(54) **TISSUE REGENERATING IMPLANTING MATERIAL**
IMPLANTATIONSMATERIAL ZUR GEWEBEREGENERATION
MATERIAU IMPLANTAIRE DE REGENERATION DE TISSU

(30) Priority: 30.07.2001 JP 2001230260
(43) Date of publication of application: 19.05.2004
(73) Proprietor: Japan Tissue Engineering Co., Ltd., Gamagori-shi, Aichi 443-0022 (JP); Ochi, Mitsuo, Hiroshima-shi, Hiroshima 731-0137 (JP); Ikada, Yoshito, Uji-shi Kyoto 611-0011 (JP)
(72) Inventor: OCHI, Mitsuo, Hiroshima-shi, Hiroshima 731-0137 (JP); IKADA, Yoshito, Uji-shi, Kyoto 611-0011 (JP); SUGAWARA, Katsura, c/o RINPIA OOYA 402, Okazaki-shi, Aichi 444-0823 (JP)
(74) Representative: Kuhnen & Wacker
(86) International application number: PCT/JP2002/007532
(87) International publication number: WO 2003/011353

(56) References cited:
- EP-A- 0 934 750
- WO-A-98/13076
- WO-A-99/65534
- WO-A1-99/65534
- DE-A1- 4 120 325
- DE-A1- 19 721 661
- JP-A- 4 005 965
- JP-A- 11 267 193
- JP-A- 2000 237 298

## Description

### Technical Field

The present invention relates to an implant material for tissue regeneration that is used to culture cells and reconstruct tissue in vivo and in vitro.

### Background Art

A number of techniques have been reported recently to culture cells in vitro and implant resulting cultured tissues in a patient. The cells may not be cultured alone, but in many cases, the cells are seeded and cultured on a carrier (base material for tissue regeneration) used as a scaffold of cell proliferation. The carrier has especially important roles to prepare tissues in a three-dimensional shape having a certain depth or height. The recently developed, attention-drawing technique implants a carrier or base material functioning as a scaffold of tissue regeneration and reproduces the tissues in vivo by taking advantage of the self-healing power. This technique is called regenerative medicine or tissue engineering.

Biocompatible materials and bioabsorbable materials are applied to the carrier or the base material for tissue regeneration. Available examples include collagen, hyaluronic acid, polyrotaxane, gelatin, fibronectin, heparin, chitin, chitosan, laminin, calcium alginate, and polyrotaxane hydrogel.

These prior art base materials for tissue regeneration have relatively low strength and difficulties in sufficiently maintaining their long, bulky, or tall shapes. The insufficient strength of the base material for tissue regeneration leads to the difficult handling in the process of culture and implantation and the poor operation ability and imposes the heavy load on the operators.

WO99/65534 discloses an implant material according to the preamble of claim 1. The document discloses a three-dimensional prosthesis in the shape of a body part comprising a first and second three-dimensional matrix and cells hold on the first matrix. It does not refer to chondrocyte and does not disclose a bone architecture comprising artificial bone or bone obtained by culturing osteoplast or osteocyte.

EP0 934 750 A2 discloses a joint implant based on a three-dimensional porous support for culturing cells in vitro or in vivo. A sealing layer is provided around the porous support, so that cells held thereon are not externally accessible.

In order to solve the problems of the prior art materials, the present invention aims to provide an easily handling implant material for tissue regeneration.

The implant material of the invention includes:
a cell-holding carrier that is formed in a
three-dimensional shape and holds a cell thereon; and a support member that is provided to surround said
cell-holding carrier and supports said cell-holding carrier in an externally accessible state,
   characterized in that the cell includes chondrocyte; and in that said implant material further comprises:
   an artificial graft (33) in a three-dimensional shape that is arranged adjacent to said cell-holding carrier; or either of osteoblast and
   osteocyte held in another part of said cell-holding carrier than the part in which the chondrocyte is hold.

In the structure of the implant material, the support member is provided to surround the cell-holding carrier and thereby effectively maintains the three-dimensional shape of the cell-holding carrier, even if the cell-holding carrier is impregnated with a cell suspension or a medium and has difficulty in maintaining its three-dimensional shape. This facilitates handling of the implant material. The support member supports the cell-holding carrier in an externally accessible state. When implant material is implanted, this arrangement enables the surrounding living tissue to gain access to the cell-holding carrier via the support member and to supply the nutrients to the cells held on the cell-holding carrier via the support member. This enhances the biocompatibility of the implant material. The implant material of this invention is thus suitable for tissue regeneration.

In the implant material of the invention, the cell-holding carrier may be a porous carrier in a three-dimensional shape (for example, carrier having a large number of pores), a gel carrier embedding cells therein, or any other equivalent carrier. The porous carrier may have a sponge, honeycomb, or any other equivalent structure, although the sponge structure is preferable. The pore diameter is not specifically restricted, as long as the pore has a size of holding a cell therein.

The implant material of the invention has an artificial graft in a three-dimensional shape that is arranged adjacent to the cell-holding carrier. The artificial graft represents a cell-free artificial material and is not specifically restricted. For example, one of bioinert materials including metals like titanium and ceramics like alumina ceramics, bioactive matrix materials like hydroxyapatite, calcium phosphate, and calcium carbonate, and other materials applicable to implants like artificial bones and artificial joints is adequately selected and used according to the desired shape and strength. In one desirable structure, the support member surrounds the artificial graft as well as the cell-holding carrier, in the externally accessible state. The artificial graft and the support member may be formed integrally of an identical material.

In the implant material of the invention, the cell includes at least one of chondrocyte, osteoblast, osteocyte.

The implant material of the invention, the cell includes chondrocyte held in one half of the cell-holding carrier and either of osteoblast and osteocyte held in the other half of the cell-holding carrier or an artificial graft (33) in a three-dimensional shape that is arranged adjacent to said cell-holding carrier. The implant material of this structure is suitable for implantation in bone/cartilage defects, since the joint has an upper layer of cartilage tissue and a lower layer of bone tissue. Here the terminology 'half' does not strictly mean '1 / 2' . The area of holding the chondrocytes and the area of holding the osteoblasts or osteocytes may be divided at any ratio, and there may be a little overlap.

When the implant material of the invention has the artificial graft in a three-dimensional shape that is arranged adjacent to the cell-holding carrier, the artificial graft may be artificial bone, and the cell may be chondrocyte. The implant material of this structure is also suitable for implantation in bone/cartilage defects, since the joint has an upper layer of cartilage tissue and a lower layer of bone tissue.

In the implant material of the invention, the support member may not completely surround the whole cell-holding carrier but may cover only part of the cell-holding carrier, as long as the support member functions to maintain the three-dimensional shape of the cell-holding carrier. The support member is preferably any of a mesh support member, a palisade support member, and a perforated plate support member. This structure enables the cell-holding carrier to be supported in an externally accessible state. In the implant material of the invention, it is preferable that at least one of the cell-holding carrier and the support member is composed of either a biocompatible material or a bioabsorbable material. It is especially preferable that both of the cell-holding carrier and the support member are composed of either the biocompatible material or the bioabsorbable material. The living body hardly recognizes an implanted base material of this composition as foreign, so that the base material of this composition is suitable for tissue regeneration. The bioabsorbable material is decomposed and absorbed after implantation and is thus especially suitable for tissue regeneration. Available examples of the biocompatible material and the bioabsorbable material are given previously. It is preferable that the support member has at least one suture thread. The implant material is fixed to the living tissue of the implantation site by means of the suture thread. The suture thread is preferably composed of the same material as that of the support member.

The implant material of the invention may be implanted after incision of the living tissue surrounding an implantation site. In order to reduce invasion against the patient, arthroscopic surgery is desirable. It is thus preferable that the implant material is formed in a specific shape available for arthroscopic surgery. For example, the implant material is formed in a columnar shape and has a diameter of 2 to 15 mm in cross section.

It is desirable that an adhesive factor, such as fibronectin, is interposed between the artificial bone and the cell-holding carrier.

### Brief Description of the Drawings

Fig. 1 is a perspective view schematically illustrating a base material for tissue regeneration in Example 1;
Fig. 2 is a perspective view schematically illustrating an implant material (bone/cartilage column) in Example 2;
Fig. 3 shows a general process of producing the implant material (bone/cartilage column) in Example 2;
Fig. 4 is a perspective view schematically illustrating an implant material (bone/cartilage column) in Example 3;
Fig. 5 shows a general process of producing the implant material (bone/cartilage column) in Example 3;

### Best Modes of Carrying Out the Invention

### [Example 1] Implant Material for Tissue Regeneration

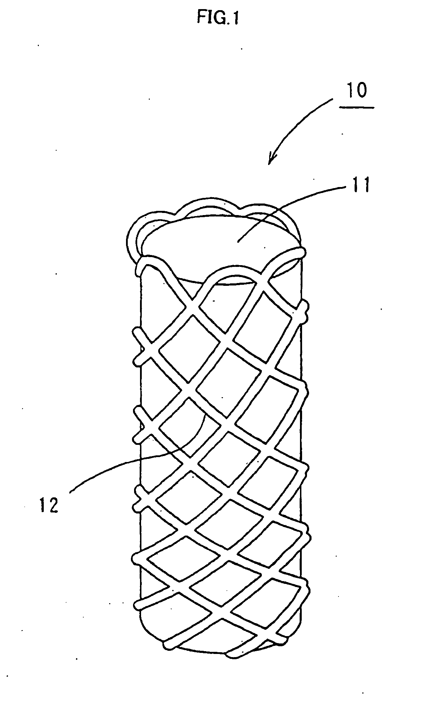

Fig. 1 is a perspective view schematically illustrating an implant material for tissue regeneration. The implant material for tissue regeneration 10 includes a collagen sponge 11 formed in a columnar shape and a mesh support member 12 provided to surround the periphery of the collagen sponge 11. The mesh support member 12 is composed of PLGA (polylactic acid-polyglycolic acid copolymer) and supports the collagen sponge 11 in an externally accessible state via its web structure. The base material for tissue regeneration 10 has an outer diameter of 6 mm and a height of 15 mm.

### [Example 2] Implant material 1

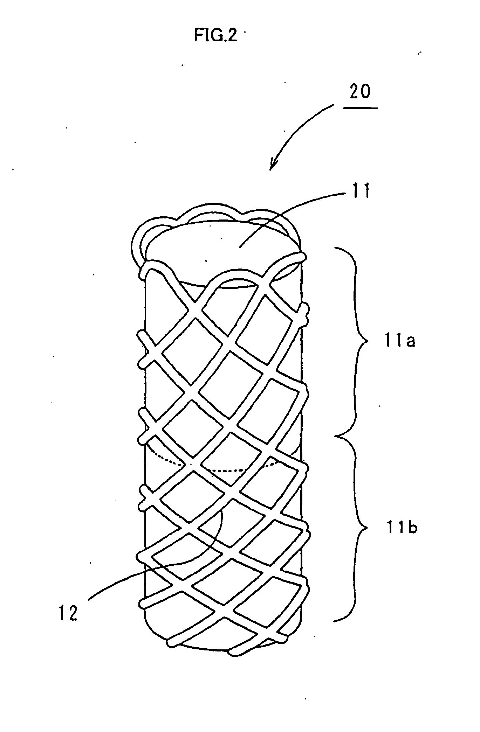

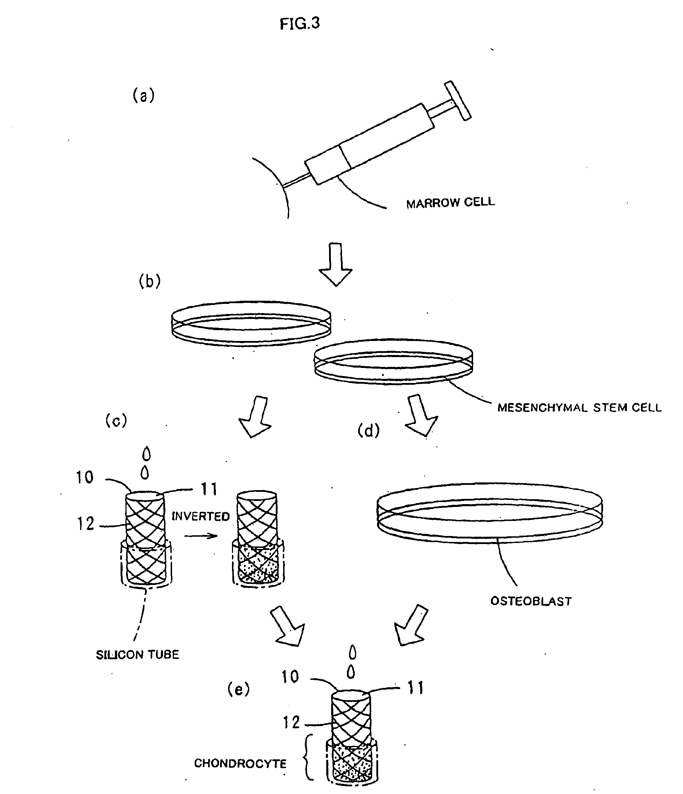

Fig. 2 is a perspective view schematically illustrating an implant material of one example. Fig. 3 shows a general process of producing the implant material of the example. This example produced an implant material (hereafter referred to as bone/cartilage column) 20 having the base material for tissue regeneration 10 of Example 1 as a scaffold of cell proliferation, chondrocytes held in a half area 11a of its collagen sponge 11, and osteoblasts held in the other half area 11b, as shown in Fig. 2. The process in general of preparing the bone/cartilage column 20 is described below with reference to Fig. 3.

The process first sucked marrow cells from the tibia of Japanese white rabbits with a syringe containing a small amount of heparin (see Fig. 3(a)), and then diluted the sucked marrow cells to 10 times with a DMEM (Dulbecco's Modified Eagle Medium) containing 10% FBS (fetal bovine serum) to prepare a cell suspension. Antibiotics were added to the DMEM. The process seeded 10 ml of the cell suspension in each 10 cm-dish and cultured the cells in an atmosphere of 5% CO₂ at 37°C for 1 week. After the 1-week culture, the culture medium was replaced. The marrow cells included blood cells and mesenchymal stem cells. The replacement of the culture medium removed only suspension blood cells and adhesion-dependent mesenchymal stem cells adhering to the bottom of the dish.

After the first replacement of the culture medium, the culture medium was replaced at intervals of every 3 to 4 days for proliferation of the mesenchymal stem cells (see Fig. 3(b)). In order to obtain a sufficient amount of cells, subculture was carried out according to the requirements. The subculture gives a sufficient amount of mesenchymal stem cells. Proliferation of the mesenchymal stem cells over the whole culture surface of the dish (to the state of confluent growth) starts differentiation of the stem cells. The proliferation was repeated until the state of sufficient but not confluent growth and the mesenchymal stem cells were kept in the undifferentiated state.

After proliferation of a sufficient amount of mesenchymal stem cells, the mesenchymal stem cells were detached from the bottom of the dish by trypsin treatment for 5 minutes. The detached mesenchymal stem cells were subjected to centrifugation at 1500 rpm for 5 minutes. The obtained pellets of the mesenchymal stem cells were suspended in a chondrocyte differentiation-inducing medium to prepare a cell suspension having the cell density of not less than 4×10⁷ cells/ml. The chondrocyte differentiation-inducing medium used here was a serum-free medium obtained by adding 10⁻⁸ M dexamethasone, 10⁻⁵ M β-glycerophosphate, 0.05 mg/ml ascorbic acid-2-phosphate, and antibiotics to a DMEM.

The implant material for tissue regeneration 10 described above was set in each sterile silicon tube (outer diameter: 10 mm, inner diameter: 6 mm, height: 7 mm) placed on a 24-well plate. Namely the silicon tube supported the base material for tissue regeneration 10 upright (see Fig. 3(c)). The process then added dropwise 100 µl of the cell suspension (mesenchymal stem cells + chondrocyte differentiation-inducing medium) prepared as discussed above onto the collagen sponge 11 of the base material for tissue regeneration 10 with an Eppendorf pipette. The dropping of the volume 100 µl caused the cell suspension to permeate into only an upper half of the collagen sponge 11 and made the mesenchymal stem cells adhere to the upper half of the collagen sponge 11, while keeping a lower half of the collagen sponge 11 free from the mesenchymal stem cells. The base material for tissue regeneration 10 in this state was inverted to face down the cell seeding area. The inverted base material for tissue regeneration 10 was supported upright by the silicon tube in the same manner as before. With elapse of 1 hour since the inversion, the mesenchymal stem cells were fixed to the base material for tissue regeneration 10. After adding of 1.5 ml of the chondrocyte differentiation-inducing medium having the above composition, the base material for tissue regeneration 10 with the mesencymal stem cells fixed thereon was cultured in an atmosphere of 5% CO₂ at 37°C for 2 weeks. The culture medium was replaced every other day. On completion of the culture, the process fixed the base material for tissue regeneration 10 with formalin, made a paraffin-embedded tissue slice, and stained the tissue slice by the technique of alcian blue staining used for staining the cartilage matrix. Observation of the stained tissue slice showed the presence of stained acidic mucopolysaccharides specifically produced by the chondrocytes. This proved adequate differentiation of the mesenchymal stem cells into the chondrocytes.

In the meanwhile, the process cultured the mesenchymal stem cells in some of the 10 cm-dishes to the state of confluent growth. After the confluent growth of the mesenchymal stem cells, the culture medium for cell proliferation was replaced with an osteoblast differentiation medium. The culture in an atmosphere of 5% CO₂ at 37°C for 2 weeks differentiated the mesenchymal stem cells into osteoblasts (see Fig. 3(d)). The osteoblast differentiation medium used here was prepared by adding 10⁻⁷ M dexamethasone, 0.15 mM ascorbic acid 2-phosphate, 1 mM pyruvic acid, 10 ng/ml rh TGF-β1 (recombinant human TGF-β), and 1/100 vol. (corresponding to 1/100 of the medium volume) of ITS premix (manufactured by Nippon Becton Dickinson Company, Ltd.) to a DMEM containing 10% FBS.

The differentiated osteoblasts were detached from the bottom of the dish by trypsin and collagenase treatment, and were mixed with antibiotics and a 10% FBS/DMEM to prepare an osteoblast suspension having the cell density of 2×10⁷ cells/ml. The osteoblast suspension thus obtained was added dropwise onto the base material for tissue regeneration 10 with the chondrocytes fixed in the half area of its collagen sponge 11. At this moment, the osteoblast suspension was dropped into the opposite half of the collagen sponge 11 where the chondrocytes held(see Fig. 3(e)). The base material for tissue regeneration 10 was stood still for 1 hour after the dropping of the osteoblast suspension. The osteoblasts were thus fixed on the collagen sponge 11. This gave the bone/cartilage column 20 having the chondrocytes held in the half area 11a of the collagen sponge 11 and the osteoblasts held in the other half area 11b as shown in Fig. 2.

After seeding of the osteoblasts, the process cultured the seeded osteoblasts in a DMEM containing 10% FBS with antibiotics added thereto to induce production of the matrix from the osteoblasts. This gave the bone/cartilage column 20 having the chondrocytes held in the half area 11a of the collagen sponge 11 and the osteocytes held in the other half area 11b.

### [Example 3] Implant material 2

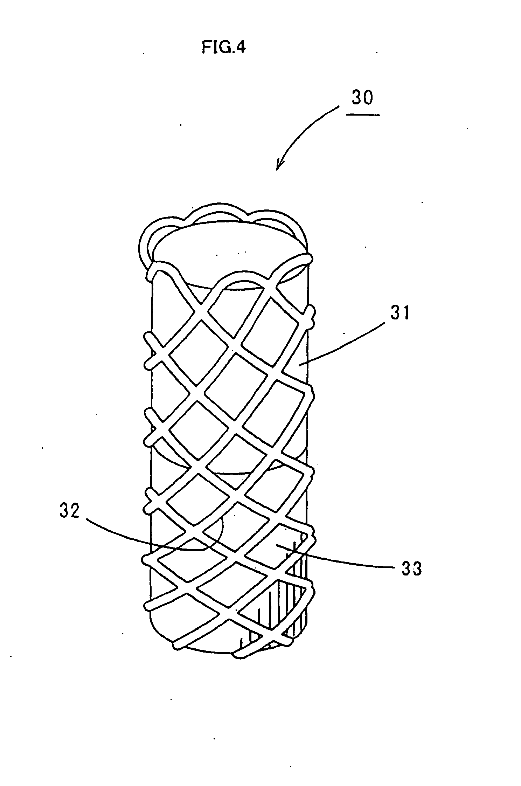

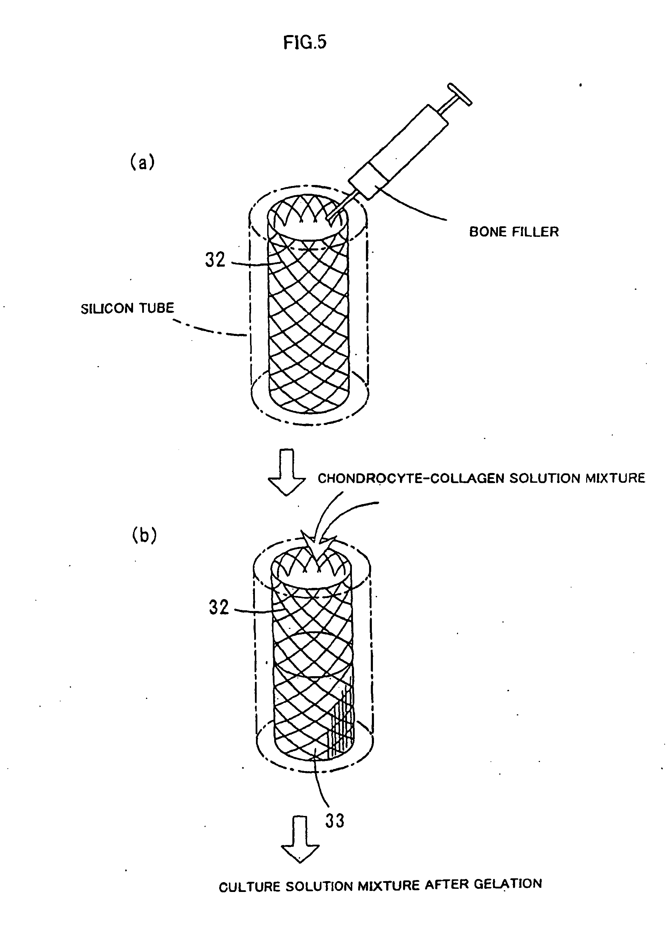

Fig. 4 is a perspective view schematically illustrating an implant material of another example. Fig. 5 shows a general process of producing the implant material of the example. This example produced a bone/cartilage column 30 as an implant material having a collagen gel 31 with chondrocytes held therein, an artificial bone 33 located below the collagen gel 31, and a mesh support member 32 formed to surround the collagen gel 31 and the artificial bone 33, as shown in Fig. 4. The process of preparing the bone/cartilage column 30 is described below with reference to Fig. 5.

The mesh support member 32 composed of PLGA (polylactic acid-polyglycolic acid copolymer) was set in each sterile silicon tube (outer diameter: 10 mm, inner diameter: 6 mm, height: 15 mm) placed on a 24-well plate. Namely the silicon tube supported the mesh support members 32 upright (see Fig. 5(a)).

The process then filled the mesh support member 32 surrounded by the silicon tube with 200 µl of a bone filler paste (trade name: Biopex manufactured by Mitsubishi Materials Corp.) containing calcium phosphate as its major component. The height of the bone filler approximated to 7 mm. The mesh support member 32 filled with the bone filler was stood still for about 10 minutes. The bone filler was then hardened to form hydroxyapatite, that is, the artificial bone 33 (see Fig. 5(b)).

In the meanwhile, the process obtained cartilage tissues from the joints of Japanese white rabbits, enzyme-treated the obtained cartilage tissues with a trypsin EDTA solution and a collagenase solution, and isolated chondrocytes. The isolated chondrocytes were washed and were mixed with a DMEM containing 10% FBS to prepare a cell suspension having the cell density of 4×10⁶ cells/ml. The process mixed the cell suspension thus obtained and 3% atelocollagen implant (manufactured by KOKEN Co., Ltd.) at a mixing rate of 1 to 1 to prepare a chondrocyte-collagen solution mixture. This mixing step diluted the cell density from 4×10⁶ cells/ml to 2×10⁶ cells/ml. The process filled 200 µl of the chondrocyte-collagen solution mixture onto the artificial bone 33 held in the mesh support member 32 described above. An adhesive factor, such as fibronectin, was placed on the artificial bone 33 in the process of filling the chondrocyte-collagen solution mixture.

The chondrocyte-collagen solution mixture on the artificial bone 33 was stood still in an atmosphere of 5% CO₂ at 37°C for 1 hour to gelate. The gel was added with a medium and was cultured for 3 weeks to induce the chondrocytes to produce the matrix. This gave a bone/cartilage column. The medium used here was a 10% FBS-DMEM containing 50 µg/ml ascorbic acid.

### [Example 4] Evaluations of Bone/Cartilage Column

The bone/cartilage column (having the cartilage tissue in one half and the bone tissue in the other half) prepared in Example 2 was actually implanted in bone/cartilage defects of rabbits for evaluation of recovery. The evaluation test procedure anaesthetized each Japanese white rabbit (27 weeks old), incised the knee joint to disjoint patella, exposed the femur, and drilled the center of patella groove to make a full-thickness defect of 5 mm in diameter and 8 mm in depth. The bone/cartilage column 20 of Example 2 was cut to the depth of the defect and was implanted in the bone/cartilage defect. The mesh of the PLGA mesh support member 12 was stitched with and fixed to the surrounding healthy cartilage tissue with a PLGA suture thread. As a control group, the base material for tissue regeneration 10 without seeding of the cells was implanted in each bone/cartilage defect and was stitched with and fixed to the surrounding healthy cartilage tissue. Each surgery site was washed with physiological saline containing antibiotics and the cut was then stitched up. Both the bone/cartilage columns and the base materials for tissue regeneration had sufficient strength and were readily handled without deformation.

After 84 days in captivity, the rabbits were sacrificed under anesthesia. The procedure cut off the knee joint of the femur including the implantation site, made a tissue specimen, and fixed the tissue specimen with formalin. The procedure then stained each tissue slice by the technique of alcian blue staining and safranin O staining and made the histological observation. In the bone/cartilage column implantation group, the defects were positively stained with alcian blue and safranin O. This revealed the recovery of the bone tissue and the cartilage tissue by means of the bone/cartilage column. In the control group, however, no sufficient recovery was observed. The recovery of the tissue by the growth of cells from the surrounding healthy tissue may be expected in the control group after the longer captivity.

Following the above procedure, the bone/cartilage column 30 (having the cartilage tissue in one half and the artificial bone in the other half) prepared in Example 3 was actually implanted in bone/cartilage defects of rabbits for evaluation of recovery. The evaluation test revealed the recovery of the defects in the implantation sites of the rabbits with the bone/cartilage column after 84 days.

### Industrial Applicability

The implant material for tissue regeneration of the invention is applicable to culture cells or reconstruct tissue in vivo and in vitro.

## Claims

1. An implant material (20; 30) comprising:
a cell-holding carrier (11; 31) that is formed in a three-dimensional shape and holds a cell thereon; and
a support member (12; 32) that is provided to surround said cell-holding carrier and supports said cell holding carrier in an externally accessible state;
**characterized in that** the cell includes chondrocyte; and **in that** said implant material further comprises:
an artificial graft (33) in a three-dimensional shape that is arranged adjacent to said cell-holding carrier; or
either of osteoblast and osteocyte held in another part of said cell-holding carrier than the part in which the chondrocyte is held.

2. An implant material in accordance with claim 1, wherein said chondrocyte is embedded in collagen gel.

3. An implant material in accordance with either one of claims 1 and 2, said implant material being formed in a columnar shape having a diameter of 2 to 15mm in cross section.

4. An implant material in accordance with any one of claims 1 through 3, wherein said support member is any of a mesh support member, a palisade support member, and a perforated plate support member.

## Patentansprüche

1. Implantatmaterial (20; 30), umfassend:
einen Zellen haltenden Träger (11; 31), der in einer dreidimensionalen Form ausgebildet ist und eine Zelle darauf hält; und
ein Trägerelement (12; 32), das um den Zellen haltenden Träger herum bereitgestellt wird und den Zellen haltenden Träger in einem von Außen zugänglichen Zustand trägt, **dadurch gekennzeichnet, dass** die Zelle einen Chondrozyten einschließt; und das Implantatmaterial ferner umfasst:
ein künstliches Implantat (33) in einer dreidimensionalen Form, das an dem Zell haltenden Träger anliegend angeordnet ist;
oder eines von beiden, einen Osteoblast und einen Osteozyten, der in einem anderen Teil des Zell haltenden Trägers gehalten wird, als in dem Teil, in dem der Chondrozyt gehalten wird.

2. Implantatmaterial nach Anspruch 1, wobei der Chondrozyt in einem Kollagengel eingebettet ist.

3. Implantatmaterial nach einem der Ansprüche 1 und 2, wobei das Implantatmaterial in Säulenform mit einem Durchmesser von 2 bis 15 mm im Querschnitt ausgebildet ist.

4. Implantatmaterial nach einem der Ansprüche 1 bis 3, wobei das Trägerelement ein beliebiges ist, ein Netz-Trägerelement, ein Palisaden-Trägerelement, und ein Lochplatten-Trägerelement.

## Revendications

1. Matériau d'implant (20, 30) comprenant :
un support de retenue de cellule (11, 31) qui est réalisé en trois dimensions et retient une cellule dessus ; et
un élément de support (12, 32) qui est réalisé pour entourer ledit support de retenue de cellule et supporte ledit support de retenue de cellule dans un état accessible de l'extérieur ;
**caractérisé en ce que** la cellule comprend un chondrocyte ; et **en ce que** ledit matériau d'implant comprend en outre :
une greffe artificielle (33) dans une forme en trois dimensions, qui est disposée de manière adjacente audit support de retenue de cellule, ou
soit un ostéoblaste et un ostéocyte maintenu dans une partie dudit support de retenue de cellule différente de celle où le chondrocyte est maintenu.

2. Matériau d'implant selon la revendication 1, dans lequel ledit chondrocyte est intégré dans un gel de collagène.

3. Matériau d'implant selon l'une quelconque des revendications 1 et 2, ledit matériau d'implant étant réalisé en une forme colonnaire ayant un diamètre de 2 à 15 mm en section transversale.

4. Matériau d'implant selon l'une quelconque des revendications 1 à 3, dans lequel ledit élément de support est l'un quelconque des éléments parmi un élément de support en maille, un élément de support de palissade, et un élément de support de plaque perforée.
